(19) Europäisches Patentamt
European Patent Office
Office européen des brevets

(11) Veröffentlichungsnummer: 0 280 167
A2

(12) # EUROPÄISCHE PATENTANMELDUNG

(21) Anmeldenummer: 88102269.3

(51) Int. Cl.4: A61K 31/645 , A61K 31/12

(22) Anmeldetag: 17.02.88

(30) Priorität: 26.02.87 DE 3706175

(43) Veröffentlichungstag der Anmeldung:
31.08.88 Patentblatt 88/35

(84) Benannte Vertragsstaaten:
AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Hoffmann, Dieter, Dr.
Im Stetefeld 6
D-3551 Lahntal(DE)
Erfinder: Kraemer, Hans Peter, Dr.
Birkenweg 16
D-3550 Marburg(DE)
Erfinder: Hermentin, Peter, Dr.
Barfüssertor 30
D-3550 Marburg(DE)
Erfinder: Gerken, Manfred, Dr.
Wannkopfstrasse 12
D-3550 Marburg(DE)
Erfinder: Kolar, Cenek, Dr.
Deutschhausstrasse 20
D-3550 Marburg(DE)

(74) Vertreter: Meyer-Dulheuer, Karl-Hermann, Dr. et al
HOECHST Aktiengesellschaft Zentrale Patentabteilung Postfach 80 03 20
D-6230 Frankfurt/Main 80(DE)

(54) **Verwendung von lipophilen Anthracyclinen zur Behandlung "sekundär" anthracyclin-resistenter Tumoren, Mittel enthaltend ein lipophiles Anthracyclin und Verfahren zu seiner Herstellung.**

(57) Es wird die Verwendung von lipophilen Anthracyclinen zur Behandlung von "sekundär" anthracyclin-resistenten Tumoren sowie ein Mittel zur Therapie von Tumoren beschrieben, die gegen eine Behandlung mit zunächst wirksamen hydrophilen Anthracyclinen resistent geworden sind. Ein solches Mittel enthält ein lipophiles Anthracyclin sowie gegebenenfalls ein primär gegen Tumoren wirksames hydrophiles Anthracyclin.

Weiterhin wird ein Verfahren zur Herstellung eines solchen Mittels beschrieben.

EP 0 280 167 A2

Xerox Copy Centre

# Verwendung von lipophilen Anthracyclinen zur Behandlung "sekundär" anthracyclin-resistenter Tumoren, Mittel enthaltend ein lipophiles Anthracyclin und Verfahren zu seiner Herstellung

Die Erfindung betrifft die Verwendung von lipophilen Anthracyclinen als Therapeutika zur Behandlung sekundär anthracyclin-resistenter Tumoren, ein Mittel, das ein lipophiles und gegebenenfalls ein hydrophiles Anthracyclinderivat enthält, sowie ein Verfahren zu seiner Herstellung. Anthracycline im Sinne der Erfindung sind Anthracycline, Anthracyclinderivate und Anthracyclin-Aglykone.

Anthracycline, besonders Doxorubicin ($^R$Adriamycin), Epirubicin oder Daunomycin besitzen bei einer Vielzahl von Tumorerkrankungen therapeutische Wirksamkeit. Nach mehrmaliger Behandlung wird jedoch eine deutliche Verringerung der antitumoralen Wirksamkeit bis hin zur Wirkungslosigkeit beobachtet.

Es ist bekannt, daß nach der Applikation von Anthracyclinen in vitro und in vivo deren intrazelluläre Konzentration in resistenten Tumorzellen im Vergleich zu sensitiven Tumorzellen erniedrigt ist. Es ist vorgeschlagen worden, durch Inhibition des Auswärtstransports die intrazelluläre Anthracyclinkonzentration zu erhöhen und damit die Wiederherstellung der Sensitivität beispiels weise gegen Doxorubicin zu bewirken. Für bestimmte Calcium-oder Calmodulin-Antagonisten sowie für ein Anthracyclin-Aglykon wurde in vitro bereits eine derartige Wirkung nachgewiesen (Proceedings, 77th Meeting of the American Association for Cancer Research, Vol. 27, 335, 1986). Die hohe Toxizität dieser Substanzen erschwert jedoch eine ausreichend hohe Dosierung in vivo, so daß bislang nur eine geringe therapeutische Wirkung erreichbar ist.

Es wurde nun überraschend gefunden, daß Aclacinomycin eine Erhöhung der $^R$Adriamycin-Aufnahme in adriamycinresistente L1210-Leukämiezellen verursacht. Nach Inkubation mit $^R$Adriamycin und gleichzeitiger Zugabe von Aclacinomycin werden vergleichbare intrazelluläre $^R$Adriamycin-Konzentrationen in adriamycin-resistenten und -sensitiven Zellen erreicht. Demgegenüber wird die $^R$Adriamycin-Aufnahme in sensitive L1210 Zellen nicht durch Aclacinomycin beeinflußt. Diese Daten zeigen, daß Aclacinomycin A ähnlich wie die Calcium-oder Calmodulin-Antagonisten in der Lage ist, durch Beeinflussung des Anthracyclintransportes die $^R$Adriamycin-Resistenz zu durchbrechen.

Es wurde weiterhin gefunden, daß eine vergleichbare Erhöhung der intrazellulären $^R$Adriamycin-Konzentration in resistenten L1210 Zellen durch die Anthracyclin-Aglykone $\beta$-Rhodomycinon, gamma-Rhodomycinon und epsilon-iso-Rhodomycinon sowie durch lipophile N-Alkyl-oder N-Benzyl-Derivate des 7-0-alpha-L-Rhodosaminyl-$\beta$-rhodomycinon erreicht wird. Characteristisch für diese Substanzen ist ihre große Lipophilie mit einem Oktanol-Wasser Verteilungskoeffizienten P größer als 100 bei Raumtemperatur (20°C). Demgegenüber haben hydrophile Anthracyclin-Derivate (P kleiner als 1) keinen Einfluß auf die $^R$Adriamycin-Aufnahme. Auch Aclacinomycin A weist eine für Anthracycline sehr hohe Lipophilie von P etwa 200 auf. Die im Proliferationstest ermittelte zytotoxische Wirkung von $^R$Adriamycin auf nicht resistente L1210 Leukämiezellen wird durch Aclacinomycin oder andere lipophile Anthracyclin-Aglykone und -derivate nur - schwach im Sinne einer additiven Wirkung beeinflußt. Demgegenüber ist die Zytotoxizität von $^R$Adriamycin auf resistente L1210 Zellen in Anwesenheit lipophiler Anthracycline stark erhöht. Einen ähnlichen Einfluß üben lipophile Anthracycline auf die cytotoxische Wirkung der zu Adriamycin kreuzresistenten Anthracycline Epirubicin und Daunomycin aus.

Lipophile Anthracyclin-Aglykone und -Derivate wie Aclacinomycin sind daher zur therapeutischen Behandlung von Tumoren geeignet, die gegen Doxorubicin, Epirubicin oder Daunomycin resistent geworden sind.

Doxorubicin, Epirubicin und Daunomycin sind hydrophile Anthracyclinderivate mit einem Verteilungskoeffizienten in Octanol/Wasser von kleiner als 10.

Gegenstand der Erfindung ist deshalb die Verwendung eines lipophilen Anthracyclins zur Herstellung eines Mittels zur Behandlung von Tumoren, die gegen eine Behandlung mit zunächst wirksamen hydrophilen Anthracyclinen resistent geworden sind.

Solche hydrophilen Anthracycline sind vor allem solche mit einem Verteilungskoeffizienten in Octanol/Wasser von bis zu 10 und besonders Doxorubicin, Epirubicin oder Daunomycin.

Gegenstand der Erfindung ist auch ein Mittel enthaltend ein lipophiles Anthracyclin und gegebenenfalls ein gegen Tumoren primär wirksames hydrophiles Anthracyclin.

Ein solches hydrophiles Anthracyclin ist besonders Doxorubicin, Epirubicin oder Daunomycin.

Ein lipophiles Anthracyclin im Sinne der Erfindung ist besonders ein solches mit einem Verteilungskoeffizienten in Octanol/Wasser von gleich oder größer als 100.

Vorzugsweise ist ein geeignetes Anthracyclin ein solches der Formel I

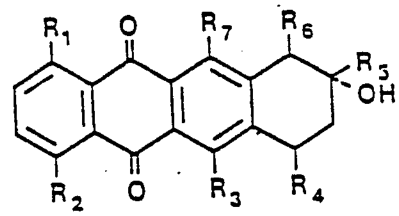

I

worin

$R_1$ Wasserstoff oder eine Hydroxygruppe,
$R_2$ Wasserstoff, eine Hydroxygruppe oder eine Methoxygruppe,
$R_3$ Wasserstoff oder eine Hydroxygruppe,
$R_4$ Wasserstoff, eine Hydroxygruppe oder eine Struktur der Formel II sind

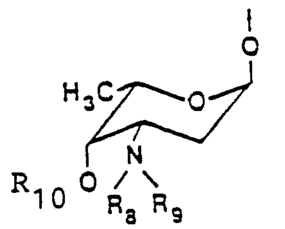

II

in der

$R_8$ und $R_9$ unabhängig voneinander Wasserstoff, ein alipathischer Rest mit bis zu 10 Kohlenstoffatomen oder $C_1$-$C_{10}$-Acyl und
$R_{10}$ Wasserstoff oder L-Cin A-alpha-1,4-L-deFuc-alpha ist, wobei im letzten Fall $R_1$ und $R_7$ Wasserstoff, $R_2$ und $R_3$ Hydroxygruppen, $R_5$ eine Ethylgruppe, $R_6$ eine Methoxycarbonylgruppe und $R_8$ und $R_9$ Methylgruppen sind,
$R_5$ eine Ethyl-, Methylcarbonyl-oder Hydroxymethylcarbonylgruppe oder $CHOHCH_3$ oder $CHOHCH_2OH$,
$R_6$ Wasserstoff, eine Hydroxygruppe oder eine Methoxycarbonylgruppe und
$R_7$ Wasserstoff oder eine Hydroxygruppe sind,
wobei die Verbindung der Formel I ausgenommen ist, in der $R_1$ und $R_7$ Wasserstoff, $R_2$, $R_3$ und $R_4$ eine Hydroxygruppe, $R_5$ Ethyl und $R_6$ $COOCH_3$ sind (Aklavinon), sowie die beiden Verbindungen in deren $R_1$ und $R_6$ Wasserstoff, $R_2$ Methoxy, $R_3$, $R_4$ und $R_7$ eine Hydroxygruppe und $R_5$ $COCH_2OH$ oder $COCH_3$ sind (Adriamycinon und Daunomycinon).

Geeignet sind beispielsweise Aclacinomycin, β-Rhodomycinon, epsilon-iso-Rhodomycinon, gamma-Rhodomycinon oder 3'-N-Alkyl-3'-N-methyl-derivate von 7-O-alpha-L-Daunosaminyl-β-rhodomycinon, die in der deutschen Patentanmeldung P 36 41 833 beschrieben sind.

Ein solches Mittel enthält von 0,1 bis 500 mg eines solchen hydrophilen Anthracyclinderivats und 0,1 bis 500 mg eines lipophilen Anthracyclinderivats pro Dosis und kg Körpergewicht und gegebenenfalls einen physiologisch unbedenklichen Träger und/oder Stabilisator und/oder Hilfsstoff.

Es wird vorzugsweise intravenös appliziert.

Gegenstand der Erfindung ist weiterhin ein Verfahren zur Herstellung eines solchen Mittels, dadurch gekennzeichnet, daß ein lipophiles Anthracyclin und gegebenenfalls ein gegen Tumoren primär wirksames hydrophiles Anthracyclinderivat gegebenenfalls mit einem physiologisch unbedenklichen Träger und/oder einem Stabilisator und/oder einem Hilfsstoff zu einem zur Therapie geeigneten Mittel verarbeitet werden.

Für diesen Zweck geeignete Verfahren sind bekannt.

Prüfmethode:

Die Züchtung von adriamycin-resistenten L1210 Zellen erfolgte durch regelmäßig wiederholte, einstündige Inkubation von sensitiven L1210 Zellen mit steigenden Konzentrationen an Adriamycin bis ein Resistenzgrad ($IC_{50}$ resistente L1210/$IC_{50}$ sensitive L1210) gegenüber Adriamycin von etwa 80 erreicht war.

Zur Bestimmung der Adriamycinaufnahme wurden sensitive oder resistente Zellen ($2 \times 10^5$/ml) in dem Zellkulturmedium RPMI 1640 für 1h bei 37°C mit Adriamycin in Gegenwart und Abwesenheit eines lipophilen Anthracyclins inkubiert. Die Zellsuspension (5 ml) wurde danach zentrifugiert (1 min, 2000 g), das

Zellpellet zweimal mit kalter phosphat-gepufferter Kochsalzlösung gewaschen und anschließend durch Zugabe von 100 µl [R]Triton X-100, 800 µl DNAse (Sigma, FRG) und 100 µl internem Standard (Daunomycin, 1 µg/ml) lysiert. Adriamycin und interner Standard wurden mit Hilfe von mit C18-Alkylresten derivatisiertem Kieselgel gefüllten Säulen (Bondelut C18, Analytichem) angereichert.

Nach Aktivierung dieser Säulen mit Methanol und Waschen mit doppelt destilliertem Wasser wurden die Proben auf die Säulen gegeben. Anschließend wurde mit doppelt destilliertem Wasser gewaschen und das gebundene Material mit 2 ml Chloroform/Methanol (2:1; v:v) eluiert. Nach dem Abdampfen des Lösungsmittelgemisches unter einem Stickstoffstrom wurden die Rückstände in jeweils 100 µl eines Gemisches aus 3 Teilen Acetonitril und 7 Teilen 20 mmol/l wässriger Phosphorsäure, pH 3 ("mobile Phase") gelöst. Die Auftrennung der Probenbestandteile erfolgte mit Hilfe der Hochdruckflüssigkeitschromatographie (20 µl Probe) über eine mit Nucleosil C18 (Partikelgrüße 5 µm) gefüllte Säule (12.5 x 0.46 cm).

Zur Detektion wurde ein Fluoreszenzdetektor bei einer Exitations-bzw. Emissionswellenlänge von 495 bzw. 590 nm verwendet. Die quantitative Analyse von Adriamycin erfolgte mit Hilfe eines Integrators durch Bestimmung der Peakflächen unter Berücksichtigung der wiedergefundenen Menge an internem Standard und Vergleich mit Proben, die bekannte Mengen an Adriamycin enthielten.

Bestimmung des Oktanol-Wasser-Verteilungskoeffizienten P

Zu 1 ml einer 2-10 µmol/l Lösung der Anthracycline in oktanol-gesättigtem Tris-Puffer, pH 7, wurde 1 ml puffer-gesättigtes Oktanol gegeben und für etwa 1 h bei Raumtemperatur geschüttelt. Die wässrige Phase wurde anschliessend entnommen und die Substanzkonzentration in einem Fluoreszenzphotometer durch Vergleich mit Proben bekannter Konzentration bestimmt. Die Konzentration in der organischen Phase wurde durch Subtraktion der in der wässrigen Phase gefundenen Konzentration von der gesamten eingesetzten Konzentration ermittelt. Der Verteilungskoeffizient wurde berechnet nach der Formel:

$$P = \frac{\text{Konzentration in Oktanol}}{\text{Konzentration in Puffer}}$$

Tabelle 1

Wirkung von Anthracyclinen auf die $^{R}$Adriamycin-Aufnahme in sensitiven und resistenten L1210 Leukämiezellen.

| Anthracyclin | P | Konzentration (µg/ml) | Adriamycinaufnahme (in % der unbehandelten Kontrolle ohne Zusatz) L1210 | resistente L1210 |
|---|---|---|---|---|
| Kontrolle (ohne Zusatz) | | | 100 | 100 |
| ACM (Aclacinomycin) | 192 | 1 | 118 | 127 |
| | | 10 | 96 | 200 |
| epsilon-Rhodomycinon | *1000 | 1 | 106 | 188 |
| | | 10 | 90 | 197 |
| epsilon-iso-Rhodomycinon | *1000 | 1 | 90 | 146 |
| | | 10 | 87 | 144 |
| ß-Rhodomycinon | 831 | 1 | 96 | 116 |
| | | 10 | 96 | 186 |
| 3'-N-Pentyl-3'-N-methyl-7-O-alpha-L-Daunosaminyl-ß-rhodomycinon | 531 | 1 | 87 | 166 |
| | | 10 | 95 | 209 |
| 3'-N-Hexyl-3'-N-methyl-7-O-alpha-L-Daunosaminyl-ß-rhodomycinon | 775 | 1 | 97 | 197 |
| | | 10 | 97 | 197 |
| Daunomycin (Kontrolle: nicht lipophiles Anthracyclin) | 4.5 | 1 | 100 | 91 |
| | | 10 | 86 | 78 |

* bedeutet "größer als"

## Ansprüche

1. Verwendung eines lipophilen Anthracyclins zur Herstellung eines Mittels zur Behandlung von Tumoren, die gegen eine Behandlung mit zunächst wirksamen hydrophilen Anthracyclinen resistent geworden sind.

2. Mittel enthaltend ein lipophiles Anthracyclin und gegebenenfalls ein gegen Tumoren primär wirksames hydrophiles Anthracyclin.

3. Mittel nach Anspruch 2, dadurch gekennzeichnet, daß das hydrophile Anthracyclin Doxorubicin, Epirubicin oder Daunomycin ist.

4. Mittel nach Anspruch 2, dadurch gekennzeichnet, daß das lipophile Anthracyclin einen Verteilungskoeffizienten in Octanol/Wasser von gleich oder größer als 100 hat.

5. Mittel nach Anspruch 2, dadurch gekennzeichnet, daß das lipophile Anthracyclin ein solches der Formel I

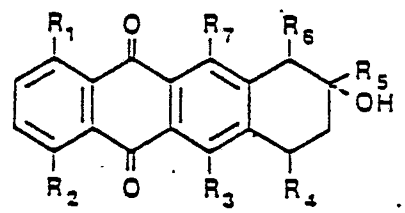

I

ist, worin
$R_1$ Wasserstoff oder eine Hydroxygruppe,
$R_2$ Wasserstoff, eine Hydroxygruppe oder eine Methoxygruppe,
$R_3$ Wasserstoff oder eine Hydroxygruppe,
$R_4$ Wasserstoff, eine Hydroxygruppe oder eine Struktur der Formel II sind

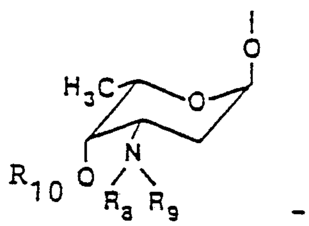

II

in der
$R_8$ und $R_9$ unabhängig voneinander Wasserstoff, ein alipathischer Rest mit bis zu 10 Kohlenstoffatomen oder $C_1$-$C_{10}$-Acyl und
$R_{10}$ Wasserstoff oder L-Cin A-alpha-1,4-L-deFuc-alpha-Fucosyl-ist, wobei im letzten Fall $R_1$ und $R_7$ Wasserstoff, $R_2$ und $R_3$ Hydroxygruppen, $R_5$ eine Ethylgruppe, $R_6$ eine Methoxycarbonyl und $R_8$ und $R_9$ Methylgruppen sind,
$R_5$ eine Ethyl-, Methylcarbonyl-oder Hydroxymethylcarbonylgruppe oder $CHOHCH_3$ oder $CHOHCH_2OH$,
$R_6$ Wasserstoff, eine Hydroxygruppe oder eine Methoxycarbonylgruppe und
$R_7$ Wasserstoff oder eine Hydroxygruppe sind,
wobei die Verbindung der Formel I ausgenommen ist, in der $R_1$ und $R_7$ Wasserstoff, $R_2$, $R_3$ und $R_4$ eine Hydroxygruppe, $R_5$ Ethyl und $R_6$ $COOCH_3$ sind (Aklavinon), sowie die beiden Verbindungen in denen $R_1$ und $R_6$ Wasserstoff, $R_2$ Methoxy, $R_3$, $R_4$ und $R_7$ eine Hydroxygruppe und $R_5$ $COCH_2OH$ oder $COCH_3$ sind (Adriamycinon und Daunomycinon).

6. Mittel nach Anspruch 2, dadurch gekennzeichnet, daß das lipophile Anthracyclin Aclacinomycin, β-Rhodomycinon, epsilon-iso-Rhodomycinon, gamma-Rhodomycin oder ein 3'-N-Alkyl-3'-N-methyl-derivat von 7-O-alpha-L-Daunosaminyl-β-rhodomycinon ist.

7. Verfahren zur Herstellung eines Mittels zur Behandlung von Tumoren dadurch gekennzeichnet, daß ein lipophiles Anthracyclin und gegebenenfalls ein gegen Tumoren primär wirksames hydrophiles Anthracyclin gegebenenfalls zusammen mit einem physiologisch unbedenklichen Träger und/oder einem Stabilisator und/oder einem Hilfsstoff zu einem zur Therapie geeigneten Mittel verarbeitet werden.

Patentansprüche für die folgenden Vertragsstaaten: ES, GR

1. Verfahren zur Herstellung eines Mittels zur Behandlung von Tumoren dadurch gekennzeichnet, daß ein lipophiles Anthracyclin und gegebenenfalls ein gegen Tumoren primär wirksames hydrophiles Anthracyclin gegebenenfalls zusammen mit einem physiologisch unbedenklichen Träger und/oder einem Stabilisator und/oder einem Hilfsstoff zu einem zur Therapie geeigneten Mittel verarbeitet werden.

2. Verfahren nach Anspruch 1, dadurch gekennzeichnet, daß das hydrophile Anthracyclin Doxorubicin, Epirubicin oder Daunomycin ist.

3. Verfahren nach Anspruch 1, dadurch gekennzeichnet, daß das lipophile Anthracyclin einen Verteilungskoeffizienten in Octanol/Wasser von gleich oder größer als 100 hat.

4. Verfahren nach Anspruch 1, dadurch gekennzeichnet, daß das lipophile Anthracyclin ein solches der Formel I

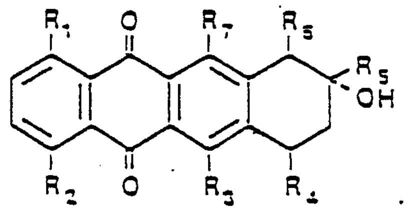

I

ist, worin

$R_1$ Wasserstoff oder eine Hydroxygruppe,

$R_2$ Wasserstoff, eine Hydroxygruppe oder eine Methoxygruppe,

$R_3$ Wasserstoff oder eine Hydroxygruppe,

$R_4$ Wasserstoff, eine Hydroxygruppe oder eine Struktur der Formel II sind

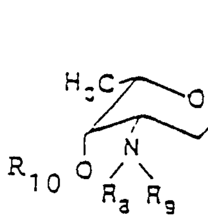

II

in der

$R_8$ und $R_9$ unabhängig voneinander Wasserstoff, ein alipathischer Rest mit bis zu 10 Kohlenstoffatomen oder $C_1$-$C_{10}$-Acyl und

$R_{10}$ Wasserstoff oder L-Cin A-alpha-1,4-L-deFuc-alpha-Fucosyl-ist, wobei im letzten Fall $R_1$ und $R_7$ Wasserstoff, $R_2$ und $R_3$ Hydroxygruppen, $R_5$ eine Ethylgruppe, $R_6$ eine Methoxycarbonyl und $R_8$ und $R_9$ Methylgruppen sind,

$R_5$ eine Ethyl-, Methylcarbonyl-oder Hydroxymethylcarbonylgruppe oder $CHOHCH_3$ oder $CHOHCH_2OH$,

$R_6$ Wasserstoff, eine Hydroxygruppe oder eine Methoxycarbonylgruppe und

$R_7$ Wasserstoff oder eine Hydroxygruppe sind,

wobei die Verbindung der Formel I ausgenommen ist, in der $R_1$ und $R_7$ Wasserstoff, $R_2$, $R_3$ und $R_4$ eine Hydroxygruppe, $R_5$ Ethyl und $R_6$ $COOCH_3$ sind (Aklavinon), sowie die beiden Verbindungen in denen $R_1$ und $R_6$ Wasserstoff, $R_2$ Methoxy, $R_3$, $R_4$ und $R_7$ eine Hydroxygruppe und $R_5$ $COCH_2OH$ oder $COCH_3$ sind (Adriamycinon und Daunomycinon).

5. Verfahren nach Anspruch 1, dadurch gekennzeichnet, daß das lipophile Anthracyclin Aclacinomycin, β-Rhodomycinon, epsilon-iso-Rhodomycinon, gamma-Rhodomycin oder ein 3'-N-Alkyl-3'-N-methyl-derivat von 7-O-alpha-L-Daunosaminyl-β-rhodomycinon ist.